# EUROPEAN PATENT APPLICATION

(11) **EP 1 634 571 A1**
(43) Date of publication of application: **15.03.2006**
(21) Application number: 05019307.7
(22) Date of filing: 06.09.2005
(51) Int. Cl.: A61K 8/37, A61Q 13/00, C07B 41/12, B01J 31/12, C11B 9/00

(54) **Transesterification process for producing salicylic esters used in perfumes**

(30) Priority: 07.09.2004 JP 2004259941
(71) Applicant: KAO CORPORATION, Chuo-ku, Tokyo (JP)
(72) Inventor: Uehara, Takefumi, Wakayama-shi Wakayama (JP); Ohno, Satoshi, Wakayama-shi Wakayama (JP); Kotachi, Shinji, Wakayama-shi Wakayama (JP); Tanaka, Shigeyoshi, Wakayama-shi Wakayama (JP)
(74) Representative: HOFFMANN EITLE

(57) **Abstract**

There is provided a noble process for producing a salicylic ester for perfumes which comprises the step of transesterifying a salicylic lower alkyl ester with an alcohol having at least one carbon atom which is located adjacent to a hydroxyl-bonded carbon atom and has one or more hydrogen atoms bonded thereto, in the presence of a tin-based catalyst. The process of the present invention enables the salicylic ester to be produced at a high yield and is free from handling problems such as precipitation of solids in a distillation residue obtained after the reaction, and the catalyst used therein is reusable.

## Description

### FIELD OF THE INVENTION

The present invention relates to a process for producing salicylic esters, and more particularly to an improved process for producing salicylic esters suitably usable as an ingredient for perfumes.

### BACKGROUND OF INVENTION

Salicylic esters have been used in various applications. In one of the applications, the salicylic esters are used for perfumes. For example, methyl salicylate, ethyl salicylate, butyl salicylate, isobutyl salicylate, pentyl salicylate, isopentyl salicylate, hexyl salicylate, cis-3-hexenyl salicylate, cyclohexyl salicylate, phenylethyl salicylate, etc., have been practically used for the purpose of perfumes.

As known in the art, the salicylic esters have been conventionally produced by the direct esterification method in which salicylic acid is directly reacted with an alcohol, and the transesterification method in which a salicylic lower alkyl ester such as methyl salicylate is reacted with an alcohol.

The salicylic lower alkyl esters such as methyl salicylate and ethyl salicylate are readily produced at a high yield even by the direct esterification reaction between salicylic acid and a corresponding alcohol. On the other hand, upon production of salicylic esters whose ester moiety is constituted of an organic group having a large number of carbon atoms such as a chain-like aliphatic group or a cyclic aliphatic group having 3 or more carbon atoms, the direct esterification reaction between salicylic acid and the corresponding alcohol proceeds only at a low esterification rate. If the esterification reaction temperature is raised to increase the esterification rate, there arise problems such as poor yield due to occurrence of undesirable side reactions. Under these circumstances, such salicylic esters have been generally produced by the transesterification method in which the salicylic lower alkyl esters such as methyl salicylate and ethyl salicylate are transesterified with the corresponding alcohol to obtain the aimed esters.

In the transesterification reactions for producing the salicylic esters, there have been generally used transesterification catalysts. For example, there are disclosed the methods using sodium methoxide as the catalyst (e.g., refer to JP 60-160040A), and the methods using potassium carbonate as the catalyst (e.g., refer to Brazilian Patent Application Laid-Open No. 8905636). However, in these conventional methods using the basic catalysts, it is difficult to suppress formation of by products, and a water-washing treatment is inevitably required to recover the aimed salicylic esters from the resultant reaction solution at a high efficiency. Besides, the processes including such a water-washing treatment tend to suffer from disadvantages such as poor yield due to loss of the discharged reaction solution, need of disposal treatment for the waste wash water, and non-reusable catalyst.

In addition, there are disclosed the methods for producing hydroxybenzoic esters using a titanium-based catalyst soluble in the reaction system (e.g., refer to French Patent Application Laid-Open No. 2733981). However, the titanium-based catalyst tends to be deactivated in the reaction system and is, therefore, difficult to reuse, resulting in high production costs. Further, there are also caused handling problems such as increased viscosity of distillation residues obtained after the reaction and precipitation of solids in the distillation residues.

### SUMMARY OF THE INVENTION

The present invention provides a process for producing salicylic esters for perfumes by transesterifying a salicylic lower alkyl ester with an alcohol having at least one carbon atom which is located adjacent to a hydroxyl-bonded carbon atom and has one or more hydrogen atoms bonded thereto, in the presence of a tin-based catalyst.

### DETAILED DESCRIPTION OF THE INVENTION

The present invention is directed to a noble process for producing a salicylic ester at a high yield which is free from handling problems such as precipitation of solids in distillation residues obtained after the reaction, and allows the catalyst to be reused.

In the process for producing the salicylic ester for perfumes according to the present invention, there is used the method in which a salicylic lower alkyl ester and a specific alcohol are subjected to a transesterification reaction in the presence of a tin-based catalyst.

The salicylic lower alkyl ester used in the present invention is a compound represented by the general formula (6): 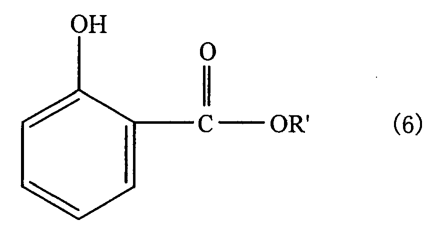 wherein R' is a linear or branched alkyl group having 1 to 4 carbon atoms.

Specific examples of the linear or branched alkyl group having 1 to 4 carbon atoms which is represented by R' in the above general formula (6), include methyl, ethyl, propyl, isopropyl, butyl, isobutyl, sec-butyl and tert-butyl.

As the salicylic lower alkyl ester, there may be generally used those esters in which the carbon number of the alkyl group contained in the ester moiety thereof is smaller than that of the alcohol used in the transesterification reaction. In view of the transesterification reactivity, among these compounds, preferred are methyl salicylate and ethyl salicylate, and more preferred is methyl salicylate.

As the alcohol to be transesterified with the salicylic lower alkyl ester, there may be used those alcohols in which at least one carbon atom located adjacent to the hydroxyl-bonded carbon atom has one or more hydrogen atoms bonded thereto.

The alcohol is not particularly limited as long as it has the above structure and is capable of producing the salicylic ester for perfumes by the transesterification reaction with the salicylic lower alkyl ester. Examples of the alcohol include a saturated or unsaturated, chain-like aliphatic or cyclic aliphatic alcohol having 3 to 20 carbon atoms, a saturated or unsaturated heterocyclic alcohol having 3 to 20 carbon atoms, and an arylalkyl alcohol having 8 to 20 carbon atoms.

In the present invention, among these alcohols, in view of the transesterification reactivity, preferred are those alcohols which are represented by the general formula (2):

R-OH (2)

wherein R is a saturated or unsaturated, chain-like aliphatic or cyclic aliphatic group having 3 to 20 carbon atoms and preferably 3 to 10 carbon atoms, in which at least one carbon atom located adjacent to the hydroxyl-bonded carbon atom has one or more hydrogen atoms bonded thereto.

Among the above alcohols, examples of the saturated chain-like aliphatic alcohol include propanol, butanol, isobutanol, 2-isopropoxy ethanol, pentanol, isopentanol, hexanol, 3-methyl-1-pentanol, heptanol, 2- or 3-heptanol, octanol, 2- or 3-octanol, 2-ethyl hexanol, nonanol, 2-nonanol, 3,5,5-trimethyl-1-hexanol, 2,6-dimethyl heptanol, 3,7-dimethyl-1-octanol, decanol, undecanol, 2-undecanol, dodecanol and 3,4,5,6,6-pentamethyl-2-heptanol (Kohinool: available from International Flavors & Fragrances Inc. (IFF)).

Specific examples of the unsaturated chain-like aliphatic alcohol include prenol (3-methyl-2-buten-1-ol), 1-penten-3-ol, cis-3-hexenol (Leaf alcohol), trans-2-hexenol, trans-3-hexenol, cis-4-hexenol, 2,4-hexadien-1-ol, 1-octen-3-ol (Matsutakeol), cis-6-nonenol, 2,6-nonadienol, 1-nonen-3-ol, 9-decenol, 1-undecenol, 4-methyl-3-decen-5-ol, 3,7-dimethoxy-7-methoxy-2-octanol, citronellol (3,7-dimethyl-6-octen-1-ol), geraniol (2-trans-3,7-dimethyl-2,6-octadien-1-ol), nerol (cis-3,7-dimethyl-2,6-octadien-1-ol), lavandulol (2-isopropenyl-5-methyl-4-hexen-1-ol), isodihydrolavandulol (2-isopropyl-5-methyl-2-hexen-1-ol), nonadyl (6,8-dimethyl-2-nonanol) and farnesol (3,7,11-trimethyl-2,6,10-dodecatrien-1-ol).

Specific examples of the saturated cyclic aliphatic alcohol include cyclopentanol, cyclohexanol, cyclohexyl methanol, 2-cyclohexyl ethanol, 4-isopropyl cyclohexanol, 4-tert-butyl cyclohexanol, 2-tert-butyl cyclohexanol, 4-(1-methylethyl)cyclohexanemethanol (Mayol: available from IFF), 5-methyl-2-isopropyl cyclohexanol (menthol), 3-thujanol (4-methyl-1-isopropylbicyclo[3.1.0]hexan-3-ol), α-fenchyl alcohol (fenchol: 1,3,3-trimethylbicyclo[2.2.1]heptan-2-ol), borneol (endo-1,7,7-trimethylbicyclo[2.2.1]heptan-2-ol: 2-camphanol), isoborneol (exo-2-camphanol), 2,2,4- and/or 2,4,4-trimethyl cyclopentanol, cycloheptanol, cyclooctanol, cyclodecanol, decahydro-β-naphthol, 2,2,6-trimethylcyclohexyl-3-hexanol, trimethyl norbornane methanol (Camekol : available from IFF): α,3,3-trimethylbicyclo[2.2.1]heptane·2·methanol) and isocamphyl cyclohexanol [3-(5,5,6-trimethylbicyclo[2.2.1]hept-2-yl)cyclohexanol].

Specific examples of the unsaturated cyclic aliphatic alcohol include 2,4-dimethyl-3-cyclohexene-1-methanol (Floralol: available from IFF), carveol (1-methyl-4-isopropenyl-6-cyclohexen-2-ol), dihydrocarveol (6-methyl-3-isopropenyl cyclohexanol), perillalcohol (dihydrocuminyl alcohol), isocyclogeraniol (2,4,6-trimethylcyclohex-3-ene-1-methanol), nopol(6,6-dimethylbicyclo[3.1.1]hept-2-ene-2-ethanol), 3,3-dimethyl-Δ²,β-norbornane-2-ethanol (Patchomint: available from IFF), cyclomethylene citronellol [3-(4-methyl-3-cyclohexenyl)butanol], ambrinol (2,5,5-trimethyl-octahydro-2-naphthol) and cyclooct-4-en-ol.

Specific examples of alcohols other than those represented by the above general formula (2) include arylalkyl alcohols such as 2-phenylethyl alcohol, hydratropalcohol (2-phenylpropyl alcohol), 2-tolylethyl alcohol (Hawthanol: available from IFF), 2-phenoxyethanol, 2-methoxy-2-phenylethyl alcohol, 1-phenyl-2-pentanol, 4-methyl-1-phenyl-2-pentanol, 3-phenylpropyl alcohol, cinnamic alcohol and 3-methyl-5-phenyl pentanol-1.

Among the above alcohols, in the present invention, there may be suitably used those alcohols having 3 to 10 carbon atoms. Examples of the preferred alcohols having 3 to 10 carbon atoms include propanol, butanol, isobutanol, pentanol, isopentanol, hexanol, 2-ethyl hexanol, octanol, 2-isopropoxyethanol, prenol (3-methyl-2-buten-1-ol), trans-2-hexenol, cis-3-hexenol, cyclopentanol, cyclohexanol, 5-methyl-2-isopropyl cyclohexanol (menthol), borneol (2-camphanol), isoborneol (exo-2-camphanol), 2,2,4- and/or 2,4,4-trimethyl cyclopentanol, 4-isopropyl cyclohexanol, cyclohexyl methanol, cyclooctanol, cyclooct-4-en-ol and 2-phenylethyl alcohol. Of these alcohols, more preferred are pentanol, isopentanol, hexanol, cis-3-hexenol and cyclohexanol.

Among these alcohols, preferred are secondary alcohols, and more preferred is cyclohexanol in view of the transesterification reactivity. In addition, preferred are unsaturated alcohols, and more preferred is cis-3-hexenol in the same aspect.

The salicylic esters produced by the transesterification reaction according to the present invention are preferably those compounds represented by the general formula (1): 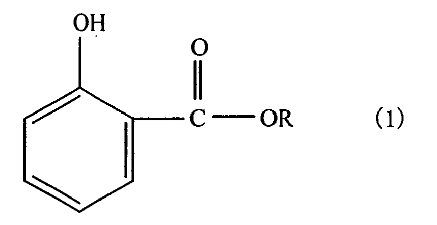 wherein R is the same as defined in the above general formula (2).

Examples of the salicylic ester represented by the above general formula (1) include propyl salicylate, butyl salicylate, isobutyl salicylate, pentyl salicylate, isopentyl salicylate, hexyl salicylate, 2-ethylhexy salicylate, octyl salicylate, 2-isopropoxyethyl salicylate, prenyl salicylate, trans-2-hexenyl salicylate, cis-3-hexenyl salicylate, cyclopentyl salicylate, cyclohexyl salicylate, esters of salicylic acid and menthol, esters of salicylic acid and borneol, esters of salicylic acid and isoborneol, 2,2,4- and/or 2,4,4-trimethylcyclopentyl salicylate, 4-isopropylcyclohexyl salicylate, cyclohexylmethyl salicylate, cyclooctyl salicylate and cyclooct-4-enyl salicylate, as well as 2-phenylethyl salicylate produced by using 2-phenylethyl alcohol as an arylalkyl alcohol. Among these salicylic esters, in the present invention, preferred are those salicylic esters in which R in the general formula (1) has 3 to 10 carbon atoms. In particular, more preferred are pentyl salicylate, isopentyl salicylate, hexyl salicylate, cis-3-hexenyl salicylate and cyclohexyl salicylate, and still more preferred are cis-3-hexenyl salicylate and cyclohexyl salicylate.

Examples of the tin-based catalyst used as the transesterification catalyst in the process for producing the salicylic ester for perfumes according to the present invention, include:
a tin compound represented by the general formula (3): 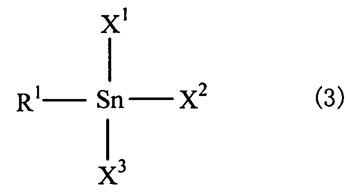
   wherein R¹ is an alkyl group or an aryl group; and X¹ to X³ are each independently an alkyl group, an aryl group, an alkoxy group, an aryloxy group, an acyloxy group, a cycloalkyl group, a hydroxyl group or a halogen atom, and a condensed product thereof;
a tin compound represented by the general formula (4): 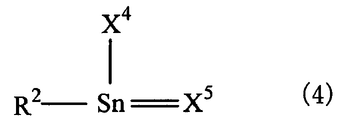
   wherein R² is an alkyl group or an aryl group; X⁴ is an alkyl group, an aryl group, an alkoxy group, an aryloxy group, an acyloxy group, a cycloalkyl group, a hydroxyl group or a halogen atom; and X⁵ is a sulfur atom or an oxygen atom, and a condensed product thereof;
a tin compound represented by the general formula (5):

   X⁶-Sn-X⁷ (5)

   wherein X⁶ and X⁷ are each independently an acyloxy group, a hydroxyl group or a halogen atom, and a condensed product thereof; and SnO.

As the alkyl group as R¹ and X¹ to X³ in the general formula (3) and as R² and X⁴ in the general formula (4), there my be used linear or branched alkyl groups having 1 to 20 carbon atoms. Specific examples of the alkyl group include methyl, ethyl, propyl, isopropyl, various butyl groups, various pentyl groups, various hexyl groups, various octyl groups, various decyl groups, various dodecyl groups, various tetradecyl groups, various hexadecyl groups and various octadecyl groups. Examples of the aryl group include aryl groups having 6 to 20 carbon atoms which may have a substituent group bonded to an aromatic ring thereof. Specific examples of the aryl group include phenyl, tolyl, xylyl and naphthyl.

Also, as the alkoxy group and the aryloxy group as X¹ to X³ in the general formula (3) and as X⁴ in the general formula (4), there may be used those alkoxy groups and aryloxy groups derived from the above-mentioned alkyl groups and aryl groups, respectively.

Further, as the acyloxy group as X¹ to X³ in the general formula (3), as X⁴ in the general formula (4) and as X⁶ and X⁷ in the general formula (5), there may be used aliphatic acyloxy groups having 2 to 20 carbon atoms, and aromatic acyloxy groups having 7 to 20 carbon atoms which may have a substituent group bonded to an aromatic ring thereof. Specific examples of the acyloxy group include acetyloxy, propionyloxy, butyryloxy, hexanoyloxy, octanoyloxy, lauroyloxy, maleoyldioxy, fumaroyldioxy, benzoyloxy, phthaloyldioxy and salicyloyloxy. As the cycloalkyl group, there may be used cycloalkyl groups having 5 to 20 carbon atoms which may have a substituent group bonded to a ring thereof. Specific examples of the cycloalkyl group include cyclopentyl, cyclohexyl, methylcyclohexyl and cyclooctyl. The halogen atom includes fluorine, chlorine, bromine and iodine. Of these halogen atoms, preferred is chlorine.

In the present invention, as the tin-based catalyst, there may be used the tin compounds represented by the above general formulae (3), (4) and (5) as well as condensed products thereof.

Specific examples of the tin-based catalyst usable in the present invention include dibutyl tin oxide, methylphenyl tin oxide, tetraethyl tin, hexaethyl ditin oxide, cyclohexahexyl ditin oxide, didodecyl tin oxide, triethyl tin hydroxide, triphenyl tin hydroxide, triisobutyl tin acetate, dibutyl tin diacetate, diphenyl tin dilaurate, monobutyl tin trichloride, dibutyl tin dichloride, tributyl tin chloride, dibutyl tin sulfide, butylhydroxy tin oxide, tin octanoate, tin oxalate, tin chloride and tin oxide. In the present invention, these tin-based catalysts may be used alone or in combination of any two or more thereof.

The amount of the tin-based catalyst used is not particularly limited, and is usually from 0.01 to 10% by weight, preferably 0.05 to 5% by weight and more preferably 0.1 to 3% by weight based on the weight of the salicylic ester as the raw material.

The amounts of the salicylic lower alkyl ester and the alcohol used in the transesterification reaction are not particularly limited, and are preferably controlled to near stoichiometric amounts in view of a good yield and costs. More specifically, the alcohol is usually used in an amount of about 0.7 to 1.7 mol, preferably 0.8 to 1.5 mol and more preferably 0.9 to 1.3 mol per mol of the salicylic lower alkyl ester.

The timing of addition of the tin-based catalyst is not particularly limited. The tin-based catalyst may be added immediately before initiation of the transesterification reaction, or may be added at an optional stage from initiation to completion of the reaction. Meanwhile, the tin-based catalyst may be previously contacted with a mixed solution containing the salicylic lower alkyl ester and the alcohol which are to be used in the transesterification reaction, and further salicylic acid, if required, before the transesterification reaction in order to enhance an initial catalytic activity thereof.

The transesterification reaction may be conducted under a pressure ranging from an ordinary pressure to a reduced pressure of about 13.3 kPa (100 mmHg). The reaction temperature may be usually selected from the range of 80 to 220°C. In the present invention, in view of the reaction rate and suppression of undesirable side reactions, the transesterification reaction is preferably conducted at a temperature not lower than 130°C but less than 180°C, and more preferably at a temperature from 140 to 170°C.

The transesterification reaction proceeds by removing lower alcohols liberated during the reaction out of the reaction system. Therefore, it is important that the reaction pressure and temperature are appropriately selected from such ranges capable of removing the lower alcohols out of the reaction system.

Although the reaction time varies depending upon kinds and amounts of catalysts used, as well as the reaction temperature and reaction pressure, the use of a reaction time of about 2 to 20 h is usually sufficient to attain a good yield. The transesterification reaction may also be performed in an atmosphere of an inert gas such as nitrogen, helium and argon, if desired.

The thus finally obtained reaction solution may be directly subjected to distillation treatment such as distillation under reduced pressure without washing treatment to recover unreacted alcohol and unreacted salicylic lower alkyl ester, and obtain the salicylic ester as the aimed product.

The distillation conditions are not particularly limited. The distillation may be conducted using an appropriate number of distillation columns. The conditions in the respective distillation columns such as a top temperature, a vacuum degree and a reflux ratio may be appropriately determined according to kinds of unreacted alcohols and unreacted salicylic lower alkyl ester to be distilled therefrom as well as the salicylic ester as the aimed product.

In the present invention, the distillation residue containing the catalyst is in a liquid state and, therefore, can be easily handled and repeatedly used as the catalyst. The present invention also provides the process for producing a salicylic ester for perfumes which comprises the steps of (a) producing the salicylic ester by the above process according to the present invention; and (b) subjecting the resultant transesterification reaction product obtained in the step (a) to distillation to remove at least the salicylic ester therefrom and obtain a distillation residue, and adding at least a salicylic lower alkyl ester and an alcohol to the distillation residue to conduct a transesterification reaction therebetween, thereby producing the salicylic ester.

More specifically, the thus obtained distillation residue was mixed with at least a salicylic lower alkyl ester and an alcohol, and the resultant mixture was subjected to the transesterification reaction in the same manner as described above, thereby enabling the salicylic ester to be produced at a high yield. The distillation residue can be repeatedly used any times as long as the catalyst contained therein maintains its catalytic activity. Meanwhile, the salicylic lower alkyl ester and the alcohol to be added upon repeated use of the catalyst are not particularly limited, and are preferably the same as used in the previous transesterification reaction process.

In the case where the salicylic ester is produced in the presence of the tin-based catalyst according to the process of the present invention, the yield of the salicylic ester upon the repeated use of the tin-based catalyst is substantially the same as that upon the previous use thereof. On the contrary, when the salicylic ester is produced in the presence of a titanium-based catalyst, the resultant distillation residue has a high viscosity and is, therefore, difficult to handle, resulting in disadvantages such as deposition of a part of solids onto a wall surface of devices used.

Also, in the conventional methods using a basic compound such as

sodium methoxide and potassium carbonate as the catalyst, the washing treatment for the finally obtained reaction solution is inevitably required, so that the yield of the product tends to be lowered, and reuse of the catalyst tends to become difficult.

Unlike the conventional methods, in the process of the present invention, various salicylic esters useful for perfumes can be efficiently produced at a high yield by the transesterification reaction, and further the process can be performed by a simple procedure, and the catalyst is reusable.

Thus, in accordance with the present invention, there is provided the process for producing the salicylic ester in an industrially useful manner at a high yield. In addition, the process of the present invention is free from problems such as precipitation of solids in the distillation residue after the reaction, thereby allowing the catalyst used in the reaction to be reused repeatedly.

The following examples further describe and demonstrate embodiments of the present invention, The examples are given only for the purpose of illustration and are not to be construed as limitations of the present invention.

### EXAMPLE 1

A four-necked glass flask were charged with 228.50 g (1.5 mol) of methyl salicylate and 165.24 g (1.65 mol) of cyclohexanol, and further 2.65 g (0.0075 mol: 0.5 mol% based on methyl salicylate) of di-n-butyl tin diacetate was added thereto under stirring. The temperature of the resultant mixture was gradually raised from 140°C to 170°C under an atmospheric pressure while distilling off methanol liberated out of the reaction system. After the temperature of the reaction solution reached 170°C, the pressure in the flask was reduced to 93.3 kPa at which the reaction solution was held under heating in a temperature range of from 160 to 170°C. Successively, while distilling off methanol liberated out of the reaction system, the contents of the flask were reacted with each other for about 2 h. The finally obtained reaction solution was subjected to gas chromatography for quantitative determination of cyclohexyl salicylate. As a result, it was confirmed that the yield of the reaction product was 86.7%.

Then, the thus obtained reaction solution was treated through ten-stage distillation columns. Specifically, first, 9.27 g of unreacted cyclohexanol was distilled off under a pressure of 1.3 kPa at a temperature of 95 to 145°C and a reflux ratio of 1, and then 11.19 g of methyl salicylate was distilled off under a pressure of 1.3 kPa at a temperature of 145 to 160°C and a reflux ratio of 10. Next, the reaction solution was further subjected to distillation under a reduced pressure of 1.3 kPa at a temperature of 160 to 164°C to obtain 254.06 g of cyclohexyl salicylate.

As a result, it was confirmed that the resultant distillation residue was in a liquid state and produced at a yield of 19.26 g (6.4% by weight based on the raw materials initially charged), and further the distillation residue exhibited a good handling property and was composed mainly of cyclohexyl salicylate containing the tin catalyst.

### EXAMPLE 2

Into 18.26 g of the distillation residue obtained in the Example 1 were added only 228.58 g (1.5 mol) of methyl salicylate and 165.59 g (1.65 mol) of cyclohexanol, and the temperature of the resultant mixture was gradually raised from 140°C to 170°C under stirring while distilling off methanol liberated therefrom. After the temperature of the reaction solution reached 170°C, the pressure of the reaction system was reduced to 93.3 kPa at which the reaction solution was held under heating in a temperature range of from 160 to 170°C. Successively, while distilling off methanol liberated, the reaction solution was reacted for about 2 h. The finally obtained reaction solution was subjected to gas chromatography for quantitative determination of cyclohexyl salicylate. As a result, it was confirmed that the yield of the reaction product was 88.8% exclusive of the amount of cyclohexyl salicylate contained in the distillation residue.

### EXAMPLE 3

A four-necked glass flask were charged with 228.20 g (1.5 mol) of methyl salicylate and 166.49 g (1.66 mol) of cis-3-hexenol, and further 2.64 g (0.0075 mol: 0.5 mol% based on methyl salicylate) of di-n-butyl tin diacetate was added thereto under stirring. The temperature of the resultant mixture was gradually raised from 140°C to 170°C under an atmospheric pressure while distilling off methanol liberated out of the reaction system. After the temperature of the reaction solution reached 170°C, the pressure in the flask was reduced to 93.3 kPa at which the reaction solution was held under heating in a temperature range of from 160 to 170°C. Successively, while distilling off methanol liberated out of the reaction system, the contents of the flask were reacted with each other for about 2 h. The finally obtained reaction solution was subjected to gas chromatography for quantitative determination of cis-3-hexenyl salicylate. As a result, it was confirmed that the yield of the reaction product was 92.2%.

Then, the thus obtained reaction solution was treated through ten-stage distillation columns. Specifically, first, 18.24 g of unreacted cis-3-hexenol was distilled off under a pressure of 1.3 kPa at a temperature of 87 to 130°C and a reflux ratio of 1, and then 14.71 g of methyl salicylate was distilled off under a pressure of 1.3 kPa at a temperature of 130 to 159°C and a reflux ratio of 1. Further, 25.52 g of initial distilled fraction containing residual methyl salicylate was distilled off under a pressure of 1.3 kPa at a temperature of 160°C and a reflux ratio of 5. Next, the reaction solution was further subjected to distillation under a reduced pressure of 1.3 kPa at a temperature of 160 to 163°C to obtain 258.88 g of cis-3-hexenyl salicylate.

As a result, it was confirmed that the resultant distillation residue was in a liquid state and produced at a yield of 15.94 g (4.7% by weight based on the raw materials initially charged), and further the distillation residue exhibited a good handling property and was composed mainly of cis-3-hexenyl salicylate containing the tin catalyst.

### EXAMPLE 4

Into 14.50 g of the distillation residue obtained in the Example 3 were added only 228.58 g (1.5 mol) of methyl salicylate and 166.47 g (1.66 mol) of cis-3-hexenol, and the temperature of the resultant mixture was gradually raised from 140°C to 170°C under stirring while distilling off methanol liberated therefrom. After the temperature of the reaction solution reached 170°C, the pressure of the reaction system was reduced to 93.3 kPa at which the reaction solution was held under heating in a temperature range of from 160 to 170°C. Successively, while distilling off methanol liberated, the reaction solution was reacted for about 2 h. The finally obtained reaction solution was subjected to gas chromatography for quantitative determination of cis-3-hexenyl salicylate. As a result, it was confirmed that the yield of the reaction product was 89.2% exclusive of the amount of cis-3-hexenyl salicylate contained in the distillation residue.

### COMPARATIVE EXAMPLE 1

The same procedure as in Example 1 was repeated except for using 2.13 g of titanium isopropoxide (0.50 mol% based on methyl salicylate) in place of di-n-butyl tin diacetate. More specifically, in the same manner as in Example 1, 228.50 g (1.5 mol) of methyl salicylate, 165.24 g (1.65 mol) of cyclohexanol and 2.13 g (0.50 mol% based on methyl salicylate) of titanium isopropoxide were charged into the flask, and subjected to transesterification reaction at a temperature of 140 to 170°C. After the temperature of the reaction solution reached 170°C, the pressure of the reaction system was reduced to 93.3 kPa while maintaining the reaction solution at a temperature of 160 to 170°C under heating, and the reaction was conducted for 2 h. As a result, it was confirmed that the yield of cyclohexyl salicylate obtained from the reaction system was 86.4%.

Further, the finally obtained reaction solution was subjected to the same procedure as in Example 1, namely, cyclohexanol and methyl salicylate were distilled off out of the reaction system, and then cyclohexyl salicylate was obtained therefrom by distillation under reduced pressure. It was conformed that 24.89 g (7.5% by weight based on the raw materials initially charged) of a distillation residue was obtained. However, the obtained distillation residue exhibited a high viscosity, and a part thereof was deposited in the form of solids onto a wall surface of the flask. The solids exhibited a poor solubility in water as well as in an organic solvent such as acetone, hexane and isopropanol. As a result, it was recognized that the distillation residue containing the titanium catalyst was deteriorated in recovery rate, and recycling of the catalyst was difficult.

### COMPARATIVE EXAMPLE 2

Using 21.88 g of the distillation residue recovered from Comparative Example 1, only methyl salicylate and cyclohexanol were charged in the same amounts as used in Example 2 and reacted with each other under the same conditions. As a result, it was confirmed that the yield of the reaction product was 78.5% which was lower than that of Comparative Example 1.

In accordance with the process of the present invention, various salicylic esters can be efficiently produced at a high yield in an industrially useful manner, and the resultant salicylic esters are useful for perfumes.

## Claims

1. A process for producing a salicylic ester for perfumes, comprising the step of transesterifying a salicylic lower alkyl ester with an alcohol having at least one carbon atom which is located adjacent to a hydroxyl-bonded carbon atom and has one or more hydrogen atoms bonded thereto, in the presence of a tin-based catalyst.

2. The process according to claim 1, wherein the salicylic ester is represented by the general formula (1): 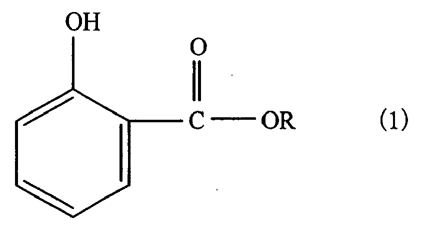
wherein R is a saturated or unsaturated, chain-like aliphatic or cyclic aliphatic group having 3 to 10 carbon atoms.

3. The process according to claim 1 or 2, wherein the salicylic ester is at least one compound selected from the group consisting of cyclohexyl salicylate, hexyl salicylate, pentyl salicylate, isopentyl salicylate and cis-3-hexenyl salicylate.

4. The process according to claim 1 or 2, wherein the salicylic ester is cyclohexyl salicylate or cis-3-hexenyl salicylate.

5. The process according to any one of claims 1 to 4, wherein the alcohol having at least one carbon atom which is located adjacent to a hydroxyl-bonded carbon atom and has one or more hydrogen atoms bonded thereto, is represented by the general formula (2):
R-OH (2)
wherein R is a saturated or unsaturated, chain-like aliphatic or cyclic aliphatic group having 3 to 10 carbon atoms.

6. The process according to any one of claims 1 to 5, wherein the alcohol having at least one carbon atom which is located adjacent to a hydroxyl-bonded carbon atom and has one or more hydrogen atoms bonded thereto, is a secondary alcohol.

7. The process according to any one of claim 1 to 5, wherein the alcohol having at least one carbon atom which is located adjacent to a hydroxyl-bonded carbon atom and has one or more hydrogen atoms bonded thereto, is a cyclohexanol.

8. The process according to any one of claim 1 to 5, wherein the alcohol having at least one carbon atom which is located adjacent to a hydroxyl-bonded carbon atom and has one or more hydrogen atoms bonded thereto, is an unsaturated alcohol.

9. The process according to any one of claim 1 to 5, wherein the alcohol having at least one carbon atom which is located adjacent to a hydroxyl-bonded carbon atom and has one or more hydrogen atoms bonded thereto, is a cis-3-hexenol.

10. The process according to any one of claims 1 to 9, wherein the tin-based catalyst is made of at least one compound selected from the group consisting of a tin compound represented by the general formula (3): 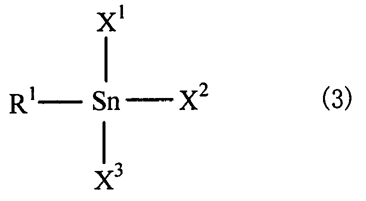 wherein R¹ is an alkyl group or an aryl group; and X¹ to X³ are each independently an alkyl group, an aryl group, an alkoxy group, an aryloxy group, an acyloxy group, a cycloalkyl group, a hydroxyl group or a halogen atom, and a condensed product thereof;
a tin compound represented by the general formula (4): 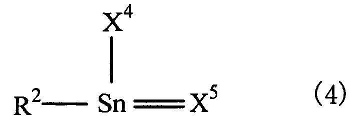
wherein R² is an alkyl group or an aryl group; X⁴ is an alkyl group, an aryl group, an alkoxy group, an aryloxy group, an acyloxy group, a cycloalkyl group, a hydroxyl group or a halogen atom; and X⁵ is a sulfur atom or an oxygen atom, and a condensed product thereof
a tin compound represented by the general formula (5):
X⁶-Sn-X⁷ (5)
wherein X⁶ and X⁷ are each independently an acyloxy group, a hydroxyl group or a halogen atom, and a condensed product thereof. and SnO.

11. The process according to any one of claims 1 to 10, wherein the transesterification reaction is conducted at a temperature not lower than 130°C but lower than 180°C.

12. A process for producing a salicylic ester for perfumes, comprising the steps of:
(a) producing the salicylic ester by the process as claimed in any one of claims 1 to 7; and
(b) subjecting the resultant transesterification reaction product obtained in the step (a) to distillation to remove at least the salicylic ester therefrom and obtain a distillation residue, and adding at least a salicylic lower alkyl ester and an alcohol to the distillation residue to conduct a transesterification reaction therebetween, thereby producing the salicylic ester.
